# EUROPEAN PATENT APPLICATION

(11) **EP 3 586 963 A1**
(43) Date of publication of application: **01.01.2020**
(21) Application number: 18180927.8
(22) Date of filing: 29.06.2018
(51) Int. Cl.: B01L 3/00, G01N 1/28, B01J 19/00, B01L 9/06

(54) **MEANS AND METHODS FOR LYSING BIOLOGICAL CELLS**

(71) Applicant: PreOmics GmbH, 82152 Plenegg (DE)
(72) Inventor: The designation of the inventor has not yet been filed
(74) Representative: Vossius & Partner Patentanwälte Rechtsanwälte mbB

(57) **Abstract**

The present invention relates to a device comprising or consisting of: (a) a vessel; (b) a magnet inside said vessel; (c) means to trigger movement of said magnet with acoustic to ultrasonic frequency, preferably ultrasonic frequencies.

## Description

The present invention relates to a device comprising or consisting of: (a) a vessel; (b) a magnet inside said vessel; (c) means to trigger movement of said magnet with acoustic to ultrasonic frequency, preferably ultrasonic frequencies.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Lysis and homogenization of cellular materials is an essential process for the analysis of biologicals. Cell lysis is used in laboratories to disrupt extracellular matrices and open cells to study their contents. The most common means for lysis are enzymatic, chemical and mechanical disruption. While enzymatic and chemical disruption methods are regularly employed, mechanical means are often used in addition due to better reliability, speed and efficiency with tough materials. Strong chemical methods may damage or modify the analytes of interest significantly while enzymes may interfere with the analytes of interest.

The most common mechanical lysis methods are repeated freeze-thawing cycles, grinding, pressure, filtration, shearing, bead milling, or sonication. The method of choice varies with the subsequent analyte of interest and the overall throughput of the method. Freeze-thawing cycles are high-throughput capable but are a soft lysis method which can easily help disrupting cell membrane but would not be capable of disrupting connective tissue efficiently. Furthermore, freeze-thawing cycles are time consuming. Grinding is often the method of choice when working with very tough materials like muscle tissue or bone but is limited by its throughput and sensitivity due to surface binding. Exhibiting pressure, such as done in the French pressure cell press, uses the property of pressure to disrupt and homogenize cells. Tough cellular materials in solution can efficiently be lyzed in large quantities, however the overall throughput is low and lysis of larger tissue materials or plant materials is nearly impossible. Filtration, similar to pressure methods, push small materials through a filter and induce rupture of the material. These methods often cause material loss due to the large filtration surface. Shearing methods such as the ULTRA-TURRAX homogenizer use cutting blades to efficiently cut and shear the material. These are the most efficient methods for most tissue materials but are also limited in throughput, may cause carry-over and are limited by their sensitivity due to unintended binding. Bead milling approaches are often used for higher throughput as multiple samples can be processed in parallel. Typically, beads are added to the sample and subsequently the sample is strongly shaken. Colliding beads cause a strong pressure difference which leads to the disruption of small materials while larger materials are being grinded simultaneously. New bead milling instruments use multidirectional shaking to efficiently cause the beads to collide and propel in any direction. The major disadvantage of bead milling approaches are the size and complexity of the instruments as well as unwanted sample loss on the large surface area of the beads.

Sonication is an efficient lysis and homogenization method for low- to medium tough materials such as cellular materials or muscle. Sonication waves are often employed to lyse, shear DNA and mix the sample at the same time. Two major types of sonication methods exist: external sonication where the ultrasound waves need to penetrate the device containing the sample, and probe-sonicators in which case a sonicator-probe needs to dip into the sample. External sonicators typically employ water-baths, are capable of higher throughput and uniform lysis but are limited by their strength as they are only indirectly in contact with the sample. Probe-sonicators can be efficient, but are of low throughput as every sample needs to be prepared sequentially and/or are not uniform if a multi-tip probe is used.

In view of the deficiencies of the state of the art, there is a need for improved means and methods of lysing biological cells.

This technical problem is solved by the subject-matter of the enclosed claims.

Accordingly, in the first aspect, the present invention relates to a device comprising or consisting of: (a) a vessel; (b) a magnet inside said vessel; (c) means to trigger movement of said magnet with acoustic to ultrasonic frequency, preferably ultrasonic frequencies.

As can be seen from the background section herein above, the device in accordance with the first aspect is preferably configured for the lysis of cellular material. The term "cellular" in this context refers to being pertinent to a cell, a cell being a basic constituent of living beings. Cells include animal and plant cells as well as tissues comprising cells, including tissues such as bone or plant material which is difficult to lyse using art-established procedures. Cells and tissues are preferably isolated, *in vitro* or *ex vivo.*

In their broadest definition, neither vessel nor magnet are particularly limited. However, feature (c) of the device of the first aspect implies that the recited means to trigger movement of the magnet have to unfold their effect inside the vessel. For example, if said means to trigger movement is or includes a magnetic field, it is understood that the material of the vessel does not (completely) shield magnetic fields.

The magnet in accordance with item (b) may consist of or comprise any sort of material which either permanently or transiently generates a magnetic field. The term "transient" refers to those materials which, when placed in a magnetic field, develop magnetic properties or show enhanced magnetic properties. The term "magnetic properties" in accordance with the present invention includes the generation of a magnetic field.

In terms of materials, both ferrimagnetic and ferromagnetic materials are preferred. Well-known ferromagnetic materials are ion, nickel and cobalt as well as their alloys. Furthermore, alloys of certain rare-earth metals display ferromagnetism. An example of such rare metal is neodymium. A particularly preferred alloy of a rare-earth metal is an alloy which comprises neodymium, iron and boron. An exemplary alloy is Nd₂Fe₁₄B.

An example of a ferrimagnetic material is magnetit (Fe₃O₄).

Since the means to trigger a movement of the magnet is preferably exterior to the vessel (see below), it follows, as stated above, that the vessel has to be made of material which permits interaction of said means (c) with the magnetic field of the magnet (b). Preferred materials include synthetic materials or plastics as well as glass. Preferred materials are described further below.

The terms "acoustic frequency" and "ultrasonic frequency" have an art-established meaning. Preferably, the term "acoustic frequency" refers to frequencies within 20 and 20,000 Hz. Above 20 kHz, frequencies are referred to as ultrasonic.

The term "ultrasonic" or "ultrasound" has no well-defined upper limit. In accordance with the invention, ultrasonic frequencies, in a preferred embodiment, are within a range of 20 kHz and 100 MHz. Further preferred ranges are given below.

As noted above, 20 kHz is generally the specific frequency value which separates acoustic from ultrasonic frequencies. For reasons of clarity, the very value of 20 kHz is considered to be embraced by the term "acoustic frequency" in accordance with the present disclosure.

Basically, the three constituents (a) to (c) are sufficient to make up a device which is capable of lysing cells. Having said that, a device in accordance with the first aspect may comprise further elements; for preferred embodiments in this respect see further below.

In the prior art, magnets inside vessels which vessels may contain biological material, are typically used for stirring. As regards art-established methods for lysing cells (reviewed in more detail in the background section herein above), bead milling is one of the preferred methods. It is considered that the action of lysis beads (the beads used in the course of bead milling) can be enhanced by stirring the solution or suspension comprising the cells to be lysed. In other words, presence of magnets in vessels comprising biological material which is to be lysed is not alien to the prior art. Yet, it is important to note that the magnets in those instances serve as stirring elements and work in conjunction with lysis beads.

The present invention is fundamentally distinct therefrom. First, it is preferred to dispense with lysis beads altogether. Quite to the contrary, for lysis in accordance with the present invention the presence of a single magnet is sufficient. Importantly, this magnet is not to be operated at those rather low frequencies which are typically used for the purpose of stirring (about 500 rpm, up to 1500 rpm, corresponding to 8.3 Hz and 25 Hz, respectively) a solution or suspension. Rather, it is to be operated at ultrasonic frequencies, or, to the extent acoustic frequencies are considered, high acoustic frequencies are preferred. For the purpose of the present invention, a high acoustic frequency is a frequency of 2 kHz up to 20 kHz.

The present inventors surprisingly discovered that such a setup - one or very few magnets; no lysis beads - yields excellent performance when used for lysing cells. As can be seen from the comparative Examples enclosed herewith, a clear outperformance of the art-established method of bead milling has been observed.

This is not only surprising, but furthermore advantageous. As noted herein above, the frequently used bead-milling approach suffers from the deficiency that, owing to numerous very small beads, significant amounts of material are lost by adsorption on the large surface of the beads. The present invention overcomes this disadvantage.

A further advantage is that a single or a few magnets can be separated easier from the lysed material than bead mills.

Given that quite simple coils work for purposes of the present invention, the design of a device in accordance with the first aspect can be very simple and cost-effective. Also, it is amenable to high throughput. This will be discussed in more detail in relation to the device in accordance with the second aspect of this invention.

In a preferred embodiment, said means in accordance with item (c) is (a) exterior to said vessel; and/or (b) at least one coil (ba)(i) comprising means for connecting it/them to a power source; and/or (ii) connected to a power source; and/or (bb) having one to one thousand, preferably one to ten, more preferably two windings.

To the extent said device comprises more than one coil, preferred are single-digit numbers of coils such as two, three, four or five coils, two coils being preferred. Altogether, one coil is most preferred. If more than one coil is present, it is preferred, but not required, that each coil has the same number of windings. The distances between coils, to the extent two or more coils are used, is not particularly limited. Generally, all coils will surround the vessel. A typical distance between two neighbouring coils is the maximal dimension of the magnet(s) in the vessel.

In a preferred configuration of the device in accordance with the first aspect, means in accordance with item (c) are exterior to said vessel. Typically, the wall of the vessel is between the magnet and said means.

The coil in accordance with item (b) of this preferred embodiment is understood to be at least one electromagnetic coil. In other words, it is a coil made of an electric conductor. When an electric current flows through the electric conductor, a magnetic field is generated. It is this magnetic field generated by the coil which triggers movement of the magnet. In most implementations, the coil is a wire made of an electric conductor made of art-established materials including metals such as copper, silver, gold, tin and alloys comprising one or more of these metals. Preferred numbers of windings are specified above. Particularly preferred are 2 windings as well as 3, 4, 5, 6, 7, 8, 9 or 10 windings. Envisaged are also coils with 20, 30, 40, 50, 60, 70, 80, 90 or 100 or several hundred windings.

It is furthermore understood that for an electric current to flow through the coil, a power source is required. Such power source may be part of the device in accordance with the first aspect. The coil is preferably equipped with means for connecting it to a power source.

Power sources may supply alternating or a direct current. In a preferred embodiment, said power source is capable of delivering pulsed current. In a preferred embodiment, the type of current flowing during the on-phase of the pulsed current is direct current.

Preferred voltages of said power source are between 10 and 60 V, preferably between 12 and 24 V. Preferred currencies are between 100 mA and 20 A, preferably between 1 A and 16 A such as 6 A.

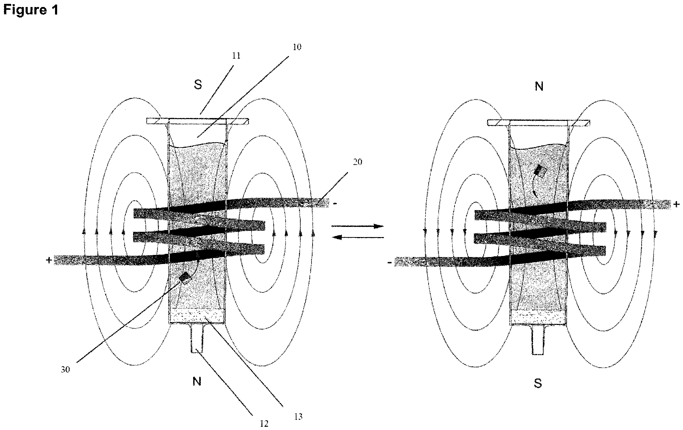

In a further preferred embodiment, said coil(s) surround(s) said vessel. More preferred is that said vessel exhibits an axis of rotational symmetry and said coil does so as well. In this case, it is preferred that the two axes of rotational symmetry align or coincide. Exemplary implementations are described in the Example and shown in Figure 1.

In a further preferred embodiment, said power source delivers (a) alternating current; and/or (b) pulsed current such as 2 to 10 times 30 to 120 sec with intervening pauses of 5 to 30 sec duration.

As noted above, pulsed current is particularly preferred. In terms of pulsed sequences, particular preference is given to a pulse length of about 60 sec. A preferred pause length is about 15 sec. A preferred number of pulses is 3.

In a further preferred embodiment, said device further comprises means for cooling said vessel. In a preferred embodiment, said device in accordance with the first aspect comprises both a power source and cooling means.

Preferred cooling means are those known in the art and include water cooling, cooling with a thermoelectric element (Peltier element) and/or compressor-based cooling.

In a further preferred embodiment, said movement, i.e. the movement of said magnet triggered by means (c) in accordance with the first aspect, is a movement in three dimensions.

"Three dimensions" refers to the three dimensions of Euclidian space. It may be defined by any three axes which are linearly independent from each other. Preferably, the three axes used to define the three dimensions are orthogonal on each other. The axes may be denoted x, y and z.

This is an important feature of the invention. Generally, the dimensions of the magnet or the few magnets are small compared to the mentions of the vessel. As a consequence, the magnet or the magnets, when triggered by means (c), perform movements in all three dimensions. This movement is free and, in preferred implementations, of similar amplitudes along the three dimensions x, y and z. Owing to the preferred size relation (magnets are small in comparison to the vessel) and the adjustment of means (c), the magnet moves freely in the three dimensions.

Related to the explanations given above, we note that the present invention does not require the dimensions of the magnet to be similar to the dimensions of the vessel, e.g. at least in one direction. While such a setup could be a means of enhancing shearing forces in a thin film of liquid between the surface of the magnet and the inner walls of the vessel, this is neither required nor preferred in accordance with the present invention. To the contrary, it is preferred that such setup is excluded.

In a further preferred embodiment, said frequency is between about 2 kHz and about 1 MHz, preferably between about 10 kHz and about 400 kHz, more preferably between about 15 kHz and about 50 kHz such as 20 kHz.

Further particularly preferred frequencies are 25 kHz, 30 kHz, 35 kHz, 40 kHz and 45 kHz.

In a further preferred embodiment of all preceding embodiments of the first aspect, including the first aspect in its broadest definition, said magnet (a) is a single magnet or a plurality of magnets, preferably from 2 to 10 magnets; (b) is a permanent magnet; (c) comprises Nd, preferably a Nd-Fe-B alloy such as Nd₂Fe₁₄B; (d) has cylindrical, cubic or spherical shape; (e) is small as compared to said vessel, wherein preferably the largest dimension of said magnet is smaller than 1/2 of the smallest dimension of said vessel; and/or (f) has a coating.

While a low number of magnets, preferably a single digit number such as 2, 3, 4, 5, 6, 7, 8 and 9 is envisaged, particular preference is given to a single magnet.

While magnetizable materials are envisaged, preference is given to a permanent magnet. Preferred materials in this respect have been discussed already further above.

Particularly preferred geometries of magnets are: a cube with about 1 mm edge length; a cylinder with a diameter of about 2 mm and a thickness of about 1 mm, and furthermore a cylinder with about 2 mm diameter and about 0.5 mm thickness (or height).

Item (e) of this preferred embodiment reiterates the notion of the magnet being small as compared to the vessel. Preferred is that the largest dimension of the magnet is smaller than 1/2 of the smallest dimension of the vessel, or smaller than 1/3 or 1/4 or 1/5 of the smallest dimension of said vessel.

Optionally, the magnet may have a coating. The coating is preferably selected from art-established plastic coatings such as polyethylene, polycarbonate, polypropylene and a synthetic fluoropolymer such as PTFE.

As noted above, it is preferred that said device does not comprise lysis beads. Having said that, in certain implementations, the use of lysis beads is envisaged. To the extent lysis beads are comprised, it is understood that these beads are typically non-magnetic beads.

In a further preferred embodiment of all preceding embodiments including the broadest definition of the first aspect of the invention, said vessel (a) is configured to receive and lyse samples of cellular material and/or viruses; (b) comprises one or more chromatographic materials, preferably one or more discs comprising or consisting of chromatographic material; (c) contains an aqueous solution or suspension; and/or (d) contains cellular material and/or viruses.

As noted above, a preferred use of the device in accordance with the first aspect is the lysis of biological samples, i.e. samples comprising cellular material. Accordingly, it is understood that it is preferred that the material of the vessel is suitable to receive samples of cellular material and withstands the lysis thereof.

In line with the above notion, it is understood that, in accordance with a further preferred embodiment, the vessel actually contains said cellular material. The cellular material may be comprised in an aqueous solution or suspension. Also, the vessel may comprise an aqueous solution or suspension which does not yet contain cellular material. An example of an aqueous solution is a buffered solution.

In a further preferred embodiment, and related to previous inventions of the present inventors, the vessel comprises one or more chromatographic materials.

In a particular preferred embodiment, said vessel is a sample preparation container in accordance with WO2014/096136, the entire disclosure contents of which is herewith incorporated by reference. As such, it is particularly preferred that said vessel is a sample preparation container for purification and/or enrichment of bio-organic compounds from cellular material, viruses and/or sub-components of said cellular material and/or viruses, the container comprising a reaction chamber and a chromatography medium; wherein said reaction chamber is for holding said cellular material, viruses and/or sub-components of said cellular material and/or viruses and is configured such that at least one of the following reactions can be performed therein: lysis, e.g. by sonication and/or boiling; chromatographic purification; reduction; alkylation; and enzymatic reactions such as proteolysis; wherein said chromatography medium is configured to purify and/or enrich said bio-organic compounds; wherein (a) said chromatography medium is located at a wall of said reaction chamber, and said wall is closed or sealed and configured to be opened for obtaining purified and/or enriched bio-organic compounds; or (b) said sample preparation container further comprises a receiving chamber for receiving said bio-organic compounds, said receiving chamber being adjacent to said chromatography medium such that said chromatography medium separates said reaction chamber from said receiving chamber, and the outer face of said receiving chamber is closed and configured to be opened for obtaining purified and/or enriched bio-organic compounds.

It is understood that the magnet(s) is/are to be placed in said sample preparation container inside said reaction chamber and in contact with said cellular material, viruses and/or sub-components of said cellular material and/or viruses.

In a further particularly preferred embodiment, said vessel is as defined in WO 2018/078106 which is herewith incorporated by reference in its entirety. In other words, in a particular preferred embodiment, said vessel is a cartridge comprising or consisting of, from top to bottom, the following elements: (a) an inlet; (b) a top volume; (c) at least one optional layer made of a first material; (d) adjacent to (b) or, if present, to (c), at least one layer of chromatographic material; (e) adjacent to (d) at least one layer made of first material; and (f) an outlet; wherein said first material is hydrophobic and porous.

It is understood that the magnet(s) is/are to be placed in said cartridge inside said top volume.

In terms of material and dimensions, preference is given to the art-established vessels obtainable from a number of manufacturers.

Preferred vessels are the vessels comprised in a PCR plate, a plate with 384 wells and a plate with 1536 wells. Also preferred are Eppendorf tubes.

In terms of metric dimensions, a well of a PCR plate has an internal diameter of about 5 mm to about 8 mm in the depth of around 15 mm. A regular 1,5 ml Eppendorf tube has an internal diameter of around 9 mm to 10 mm and a depth of around 38 mm. As known in the art, these Eppendorf tubes have a conical part at the bottom. A further preferred vessel is a 2 mm-Eppendorf tube which has an internal diameter of about 9 mm to about 10 mm and a depth of about 38 mm. Deviant from the 1.5 mm-Eppendorf tube it has a less conical shape. A well in a 384 well plate has an internal diameter of about 3 mm and a depth of about 9 mm to 10 mm. A well in 1536-well plate has an internal diameter of about 1 mm to 1.7 mm and a depth of about 5 mm.

Further types of preferred vessels are cartridges. Cartridges may be filled with chromatographic material, preferably in accordance with the two above cited patent applications, but do not have to be so. Preferred cartridges are cartridges with 1, 3, 6, 15 or 25 ml volume. In terms of metric dimensions, a 1 ml cartridge has typically an internal diameter of about 7 mm and a depth of about 5 cm. Internal diameters and depths for 3, 6, 15 and 25 ml cartridges are as follows: about 9 mm and about 6 cm, about 12 mm and about 6.5 cm, about 15 mm and about 8 cm, and about 20 mm and about 8.5 cm, respectively.

In preferred embodiments, said vessel comprises a lid. Said lid may be made of the same material as the wall material of the vessel. The lid may be a screw top. It may also be a clip closure. Furthermore, a rubber lid is also envisaged.

In a further preferred embodiment, said vessel is an array of vessels such as a microtiter plate, each vessel of said array of vessels comprising a magnet or a plurality of magnets.

Arrays of vessels are art-established and available from several manufacturers. Established formats are microwell plates with 96 wells, 384 or 1536 wells.

Related to the first aspect, the present invention provides in the second aspect a device comprising or consisting of (a) a plate with an array of holes configured to receive an array of vessels; (b) an array of coils, wherein at least two holes, preferably each hole is surrounded by at least one coil.

This aspect is specifically designed for high throughput applications.

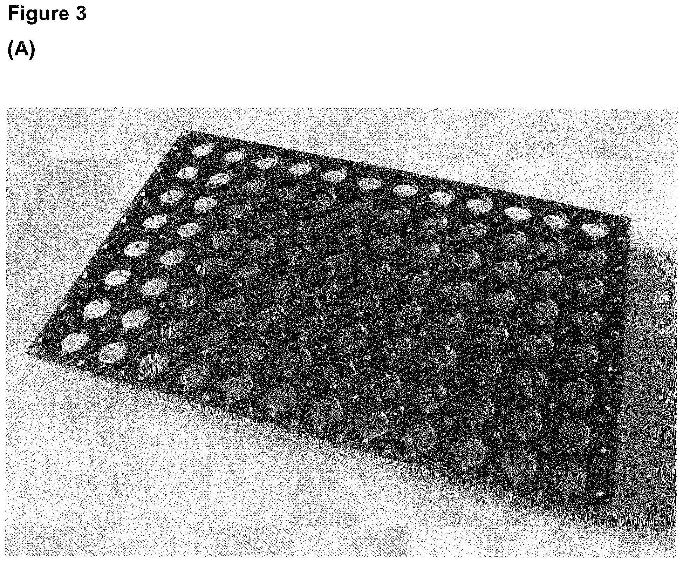

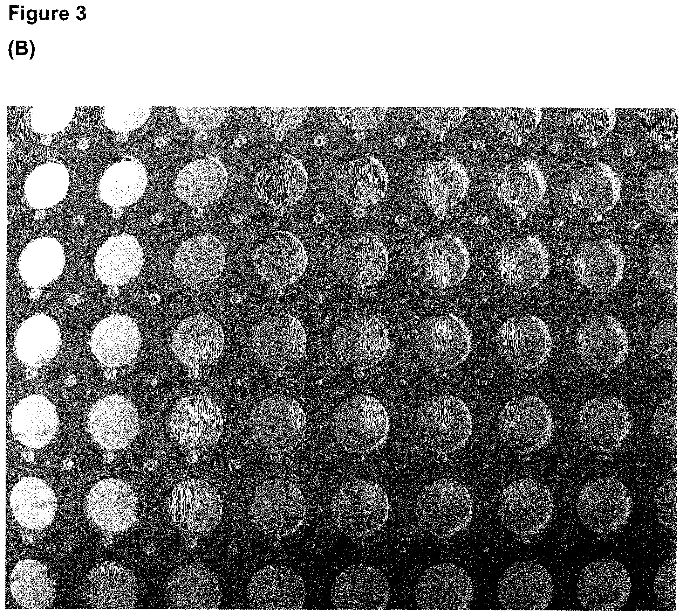

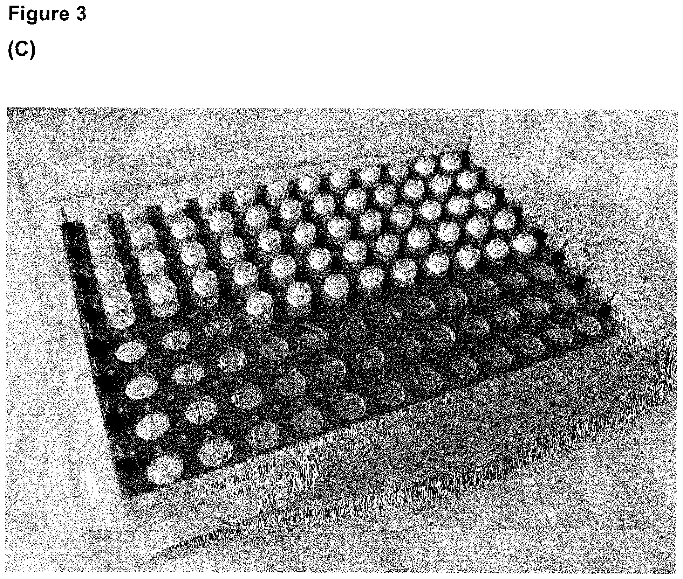

In a preferred embodiment, said device is a circuit board with said coils being printed thereon. In other words, the device in accordance with the second aspect can be conveniently manufactured. The manufacture benefits from the state of the art in the field of manufacturing circuit boards. An exemplary circuit board is shown in Figure 3. In case of more than one coil surrounding at leat two holes, preferably each hole, the printing process may comprise a plurality of steps, for example including a step of printing first coils, a step of printing a separating layer, and a step of printing second coils.

Obviously, the device in accordance with the second aspect may furthermore comprise an array of vessels. Preferably, each of said vessels comprises one or more magnets. To the extent vessels and magnets are present in the device in accordance with the second aspect, this also amounts to a preferred embodiment of the device of the first aspect.

To the extent applicable, preferred embodiments of the first aspect define preferred embodiments of the second aspect.

Related to the device of the second aspect, the present invention also provides, in a third aspect, a method of manufacturing the device of the second aspect, said method comprising printing an array of coils onto a circuit board, said array preferably comprising or consisting of 96, 384 or 1536 coils. To the extent more than one such as two coils per hole are envisaged, the number of coils would be 192, 768 or 3072, respectively.

Printing coils on a circuit board is preferably done with art-established methods. Such methods are described, for example, in Printed Circuit Boards. Design, Fabrication, and Assembly (McGraw-Hill Electronic Engineering; ISBN 0071464204).

In a fourth aspect, the present invention provides a method of performing lysis of cellular material and/or of viruses, said method comprising (a) providing a device of the first aspect, wherein said vessel(s) comprise(s) (a) sample(s) of said cellular material and/or said viruses; (b) triggering movement of the magnet(s) comprised in said device, thereby performing lysis of said cellular material or of said viruses.

As discussed herein above, the devices in accordance with the present invention are suitable for performing lysis of cellular material and viruses. This is the subject of the method of the fourth aspect.

Related thereto, in a fifth aspect, the present invention provides a method of performing lysis of cellular material and/or of viruses, said method comprising (a) providing a device of the second aspect; (b) combining said device with an array of vessels such that each vessel is surrounded by at least one coil, wherein each vessel comprises a magnet and a sample of cellular material; (c) triggering movement of the magnets comprised in said vessels; thereby performing lysis of said cellular material or of said viruses.

In essence, this is the counterpart of the method of the fourth aspect, while requiring the need of a device in accordance with the second aspect. In other words, this is the high throughput implementation of the method of performing lysis of cellular material or of viruses.

Methods of performing lysis of cellular material in accordance with the invention have been reduced to practice; see the Examples enclosed herewith. Surprisingly, outstanding performance has been observed in that the amount of proteins detectable after lysis is significantly higher when using means and methods of the present invention when compared to the art-established lysis method of bead milling.

We note that outstanding performances not only observed with regard to proteins as analytes, but also with regard to nucleic acids such as DNA.

In preferred embodiments of the methods of the fourth and fifth aspect, to the extent a method of the fourth aspect makes use of a device with a coil, said movement is triggered by an alternating and/or pulsed current flowing said coil(s).

Details on the parameters of alternating and pulsed current are given herein above.

Preferred frequencies of alternating current are between 10 Hz and 100 Hz such as 50 Hz. Also, the alternating current may be pulsed. Preferred pulse schemes are those disclosed in relation to direct current.

In a sixth aspect, the present invention provides the use of a device of the first or the second aspect of the invention for lysis of cellular material and/or of viruses.

In preferred embodiments of the methods of the fourth and fifth aspect as well as of the use in accordance with the sixth aspect, said cellular material is selected from tissue such as bone and plant material.

It is known in the art that bone as well as plant material are particularly difficult to lyse. Surprisingly, when using means and methods of the present invention, excellent performance is also observed with regard to these rather difficult materials.

In a further preferred embodiment of methods and uses of the invention, the protein yield from a given amount of a given sample is at least 1.5-fold, at least 2-fold or at least 3-fold the protein yield obtained when using bead milling.

In a seventh aspect, the present invention provides a kit comprising or consisting of: (a) a vessel; (b) a magnet; (c) means to trigger ultrasonic movement of said magnet when placed inside said vessel.

Related thereto, the present invention, in an eighth aspect, provides a kit comprising or consisting of: (a) an array of vessels; (b) an array of coils configured to receive the vessels of said array of vessels; (c) a plurality of magnets the number of which matches that of vessels in said array of vessels.

In preferred embodiments of the kits of the invention, the kit further comprises a manual with instructions to perform the methods of performing lysis of cellular material in accordance with the present invention.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The Figures show:
- **Figure 1:**: Schematic drawing of a device in accordance with the present invention. Two opposite orientations of the external magnetic field generated by a coil (20) are illustrated. The vessel (10) is equipped with chromatographic material (13). Also, it is equipped with an inlet (11) and an outlet (12). The magnet is depicted as a small square (30) with north pole and south pole. Plus and minus signs at the end of the coil indicates the connection to a power source (40) which is not shown in the drawing.

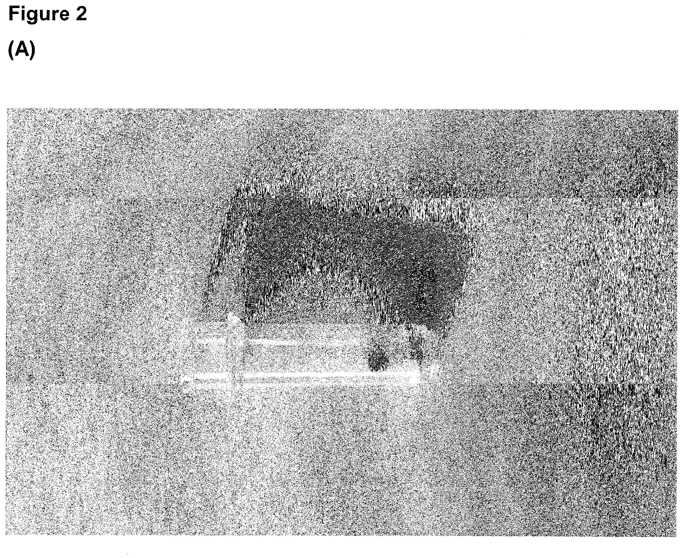

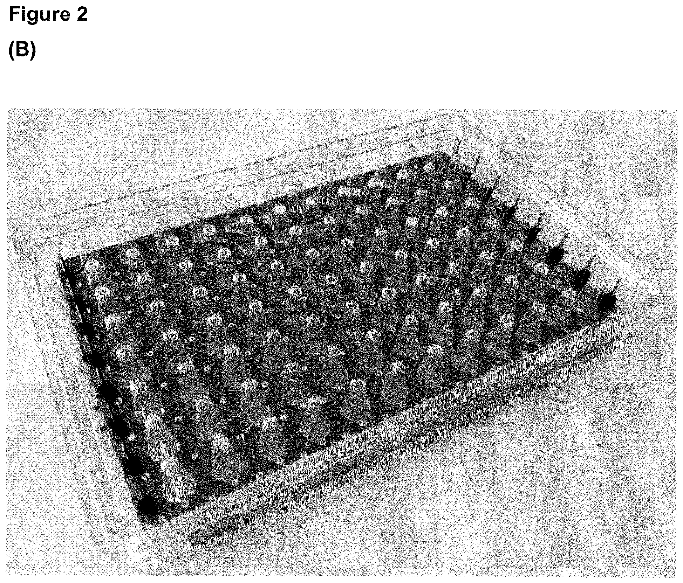

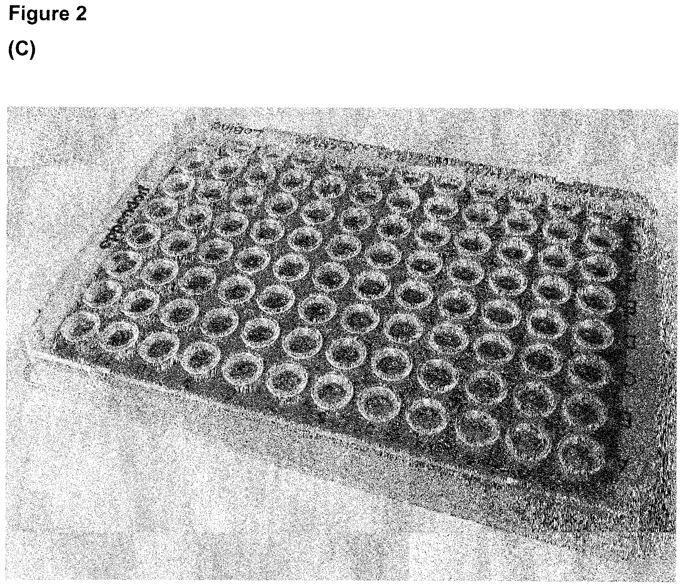

- **Figure 2:**: Figure 2(A) shows an exemplary vessel with a magnet inside. Figures 2(B) and 2(C) show arrays of vessels, designed for high throughput implementation of the methods of the present invention.
- **Figure 3:**: Figures 3(A) and 3(B) show different views of a device in accordance with the second aspect of the invention, more specifically a circuit board with printed coils thereon. The format is the 8 x 12 format of microtiter plates. In Figure 3(C), part of the holes of the circuit board are occupied with vessels.

The Examples illustrate the invention.

### Example 1

### Lysis

Triplicate test lysing and homogenizing muscle from *S. domesticus* in lysis buffer such as 0.1-10% w/v sodium deoxycholate buffer, optionally comprising detergents, demonstrated a better performance and sensitivity using a prototype setup compared to a well-established bead milling system employing the same lysis time and concentrations (Table 1). The results showed a relative 57% increase of protein extraction while using only 1/10^{th} of starting material.

Table 1: Preliminary comparison using the FastPrep Bead milling system (Bead mill 1-3) and a prototype set-up with a spool of 26 windings and a frequency of 2kHz (Magnet 1-3). Both method were used with three iterations of 60 sec. lysis and 15 sec. pauses.

| | *Bead mill 1* | *Bead mill 2* | *Bead mill 3* | *Magnet 1* | *Magnet 2* | *Magnet 3* |
|---|---|---|---|---|---|---|
| *Starting amount (mg)* | 100 | 104 | 112 | 8 | 9 | 20 |
| *Lysis buffer (µl)* | 1000 | 1040 | 1120 | 80 | 90 | 200 |
| *Protein conc. after lysis (mg*/*ml)* | 1,4 | 1,5 | 1,4 | 2,2 | 2,2 | 3,7 |

Additional tests revealed that the performance of a coil with a single winding demonstrated comparable performance as 2, 4, 8, 16 or 26 windings. Highly homogenization of grass leaves and heart muscle was also observed employing the testing platform.

## Claims

1. A device comprising or consisting of:
(a) a vessel;
(b) a magnet inside said vessel;
(c) means to trigger movement of said magnet with acoustic to ultrasonic frequency, preferably ultrasonic frequencies.

2. The device of claim 1, wherein said means is
(a) exterior to said vessel; and/or
(b) at least one coil
(ba)
(i) comprising means for connecting it/them to a power source; and/or
(ii) connected to a power source;
and/or
(bb) having one to one thousand, preferably one to ten, more preferably two windings.

3. The device of claim 2, wherein said coil(s) surround(s) said vessel.

4. The device of claim 2 or 3, wherein said power source delivers
(a) alternating current; and/or
(b) pulsed current such as 2 to 10 times 30 to 120 sec with intervening pauses of 5 to 30 sec duration.

5. The device of any one of the preceding claims, wherein said frequency is between about 2 kHz and about 1 MHz, preferably between about 10 kHz and about 400 kHz, more preferably between about 15 kHz and about 50 kHz such as 20 kHz.

6. The device of any one of the preceding claims, wherein said device does not comprise lysis beads.

7. A device comprising or consisting of
(a) a plate with an array of holes configured to receive an array of vessels;
(b) an array of coils, wherein at least two holes, preferably each hole is surrounded by at least one coil.

8. The device of claim 7, wherein said device is a circuit board with said coils being printed thereon.

9. A method of manufacturing the device of claim 8, said method comprising printing an array of coils onto a circuit board, said array preferably comprising or consisting of 96, 384 or 1536 coils.

10. A method of performing lysis of cellular material and/or viruses, said method comprising
(a) providing a device of any one of claims 1 to 6, wherein said vessel(s) comprise(s)
(a) sample(s) of said cellular material and/or viruses;
(b) triggering movement of the magnet(s) comprised in said device,
thereby performing lysis of said cellular material or of said viruses.

11. A method of performing lysis of cellular material and/or of viruses, said method comprising
(a) providing a device of claim 7 or 8;
(b) combining said device with an array of vessels such that each vessel is surrounded by at least one coil, wherein each vessel comprises a magnet and a sample of cellular material;
(c) triggering movement of the magnets comprised in said vessels;
thereby performing lysis of said cellular material or of said viruses.

12. Use of the device of any one of claims 1 to 8 for lysis of cellular material and/or of viruses.

13. The method of claim 10 or 11 or the use of claim 12, wherein said cellular material is selected from tissue such as bone and plant material.

14. A kit comprising or consisting of:
(a) a vessel;
(b) a magnet;
(c) means to trigger ultrasonic movement of said magnet when placed inside said vessel.

15. A kit comprising or consisting of:
(a) an array of vessels;
(b) an array of coils configured to receive the vessels of said array of vessels;
(c) a plurality of magnets the number of which matches that of vessels in said array of vessels.
